# EUROPEAN PATENT SPECIFICATION

(11) **EP 3 615 144 B1**
(45) Date of publication and mention of the grant of the patent: **30.03.2022**
(21) Application number: 18724080.9
(22) Date of filing: 25.04.2018
(51) Int. Cl.: A61P 31/00, A61K 33/00, A61P 41/00

(54) **METHOD FOR TREATMENT AND PREVENTION OF BIOFILM FORMATION DURING BREAST AUGMENTATION PROCEDURES**
VERFAHREN ZUR BEHANDLUNG UND PRÄVENTION VON BIOFILMAUSBILDUNG BEI BRUSTVERGRÖSSERUNGSVERFAHREN
MÉTHODE DE TRAITEMENT ET DE PRÉVENTION DE FORMATION DE BIOFILM PENDANT DES PROCÉDURES D'AUGMENTATION MAMMAIRE

(30) Priority: 25.04.2017 US 201762490025 P
(43) Date of publication of application: 04.03.2020
(73) Proprietor: Phase One Health, LLC, Nashville, TN 37228-1403 (US)
(72) Inventor: PORTER, R. Stephen, Nashville, TN 37228-1403 (US); FISHER, Jack, Nashville, TN 37228-1403 (US); RODEWALD, Albert, Nashville, TN 37228-1403 (US)
(74) Representative: Lucas, Phillip Brian
(86) International application number: PCT/US2018/029459
(87) International publication number: WO 2018/200739

(56) References cited:
- WO-A2-2013/103758
- HONGHUA HU ET AL: "Chronic Biofilm Infection in Breast Implants Is Associated with an Increased T-Cell Lymphocytic Infiltrate : Implications for Breast Implant-Associated Lymphoma", PLASTIC AND RECONSTRUCTIVE SURGERY., vol. 135, no. 2, 1 February 2015 (2015-02-01), pages 319-329, XP55489355, US ISSN: 0032-1052, DOI: 10.1097/PRS.0000000000000886
- DRAGANA AJDIC ET AL: "The Relationship of Bacterial Biofilms and Capsular Contracture in Breast Implants", AESTHETIC SURGERY JOURNAL, vol. 36, no. 3, 18 February 2016 (2016-02-18), pages 297-309, XP55489354, US ISSN: 1090-820X, DOI: 10.1093/asj/sjv177
- GIORDANO SALVATORE; PELTONIEMI HILKKA; LILIUS PETER; SALMI ASKO: "Povidone-iodine combined with antibiotic topical irrigation to reduce capsular contracture in cosmetic breast augmentation: a comparative study", AESTHETIC SURGERY JOURNAL, vol. 33, no. 5, 2013, - 2013, pages 675-680, XP002782746,

## Description

### CROSS-REFERENCE TO RELATED APPLICATIONS

This application claims the benefit of priority to U.S. Provisional Application No. 62/490,025, filed on April 25, 2017.

### TECHNICAL FIELD

Any reference to a medical method of treatment or prevention is to be regarded as referring to an agent for use in a medical method of prevention and treatment,.

The present disclosure relates generally to methods for treating or preventing the formation of bacterial biofilms during breast augmentation surgery using a hypochlorous acid solution. Furthermore, the disclosure provides methods for treating or preventing *Ralstonia Pickettii* infection using a hypochlorous acid solution. The present methods advantageously also prevent capsular contracture and anaplastic large-cell lymphoma associated with breast augmentation surgery.

### BACKGROUND OF THE DISCLOSURE

There is positive association between breast implants and anaplastic large-cell lymphoma (ALCL), a subtype of CD4+, T-cell lymphoma, and correlatively associated *Ralstonia Pickettii* infections and their antigenic stimulus. *In situ* contamination of breast implants with bacterial biofilm results in the development of capsular contracture, distortion, and pain, corroborated by animal and human studies, often necessitating revision surgery. Implants with a textured surface support a higher bacterial load with a linear relationship between the number of bacteria and lymphocytic hyperplasia on infected breast implants. Interestingly, textured implants are more frequently implicated in patients that develop ALCL. And finally, breast implant-associated ALCL surgically removed samples have significant contamination with bacterial biofilm, which provides further evidence supporting an infective etiologic contribution to ALCL. Previous investigators have found universally high levels of bacteria and bacterial biofilm on ALCL capsule specimens, and a surprising preponderance of *Ralstonia pickettii* as the dominant species. (Honghua Hu, et al. Bacterial Biofilm Infection Detected in Breast Implant Associated Anaplastic Large-Cell Lymphoma. Plast Reconstr Surg. 2016; 137(6):1659-1669.)

*Ralstonia pickettii* is a waterborne bacterium that can survive and grow in various water sources, and is a ubiquitous soil and environmental organism. *Ralstonia pickettii* is an emerging pathogen in hospital settings. *Ralstonia pickettii* may be introduced during surgical procedures by contaminated saline wound wash solutions (0.9% NS). These solutions are frequently passed through 0.22 µ filters, which do not completely remove bacterial contamination.

Breast implant-associated ALCL is heavily, if not totally, associated with textured implantable devices; however, there are many more cases in United States, where virtually all breast augmentations are with smooth implants. ALCL can occur in breast augmentation performed for either aesthetic or reconstructive purposes.

Biofilm is composed of bacteria embedded in a slimy, protective mucopolysaccharide glycocalyx. The biofilm is formed when a group of microorganisms stick to each other and become embedded within a self-produced matrix of extracellular polymeric substance composed of extracellular DNA, polysaccharides, and proteins. Biofilms generally form on solid substrates in an aqueous environment. Bacteria living in a biofilm usually have significantly different properties from free-floating bacteria of the same species, as the dense and protected environment of the film allows them to cooperate and interact in various ways. One benefit of this environment is increased resistance to detergents and antibiotics, as the dense extracellular matrix and the outer layer of cells protect the interior of the community.

The active ingredient in PhaseOne^{™} is hypochlorous acid (0.025%) has rapid and broad-spectrum antimicrobial activity against clinically relevant microorganisms. PhaseOne is a wound irrigant, and is fully capable of rapidly inactivating all groups of Gram-negative, Gram-positive, yeast and fungi including *S. aureus,* MRSA, *E. faecium,* VRE, *Acanthamoeba polyphaga* cysts, *Acinetobacter baumannii* and *bacillis anthracis* spores. Hypochlorous acid is reactive (binds and inactivates peptides, proteins, interleukins, enzymes (collagenase), endotoxins, exotoxins), and thus is not persistent within a wound environment. Thus, systemic absorption and systemic toxicity are expected to be insignificant.

A recent *in vitro* study demonstrates that many wound and skin cleansers routinely used may be toxic to fibroblasts, one of the key cells in wound repair, and suggests that these cleansers might also be toxic to other cells. When diluted to "cell safe" concentrations, most of the cleansers lost antibacterial activity as reflected by the length of time needed to reduce the growth of *S. aureus.* Although there is not a well-defined rule to quantify the relationship between *in vitro* cell toxicity of a skin/ wound cleanser and effects on healing wounds, it has been shown that *in vitro* cell toxicity correlates with retardation of healing. For example, application of SAF-Clens^{™} AF and Shur-Clens^{®} into a full-thickness guinea pig dorsum skin wounds resulted in a healing process that did not differ from healing in wounds in which saline was applied. Betadine^{®} Surgical Scrub resulted in significantly slower dermal and epidermal healing. These findings correlate with the results of in vitro fibroblast model where SAF-Clens^{™} AF and Shur-Clens^{®} were found to be non-toxic to fibroblasts at commercial concentration, while povidone-iodine (Betadine^{®} Surgical Scrub) showed the highest cytotoxicity.

Hypochlorous acid is a naturally occurring well-known broad-spectrum, fast-acting antimicrobial agent produced by neutrophils and monocytes as part of the innate immune system's response to infection. In addition to being able to directly penetrate bacteria, spores and amoeba cysts, hypochlorous acid has been shown to disrupt biofilm. Hypochlorous acid has been described as 80-100 times more potent as a germicide than the equivalent molar ratio of hypochlorite anion. This is because pure hypochlorous acid as an uncharged species can penetrate microbial cells and spore walls while the charged hypochlorite anion cannot penetrate cell walls. Previous reports clearly show that hypochlorous acid has broad-spectrum antibacterial activity against Gram-positive and Gram-negative pathogens including drug-resistant bacteria such as MRSA.

Capsular contracture is the most common complication following primary augmentation mammoplasty. It remains poorly understood but is attributed to subclinical infection, immunologic response to breast implants, and chronic inflammatory changes caused by the presence of the implants. The infectious theory of contracture has led to the practice of irrigating implant pockets with a triple antibiotic solution, (1 g of cefazolin, 80 mg of gentamicin, 50,000 IU of bacitracin, and 500 mL of normal saline) betadine (povidone-iodine), or chlorhexidine. Nevertheless, evidence that these irrigation procedures are reducing rates of contracture and other negative sequelae of breast augmentation is lacking.

Accordingly, there is a need for methods for preventing or treating bacterial infections or bacterial biofilms in a breast augmentation surgery. More specifically, there is a need for treating or preventing *Ralstonia Pickettii* infection. Finally, there is need for methods for preventing capsular contracture and anaplastic large-cell lymphoma associated with breast augmentation surgery. The present disclosure addresses these needs.

### SUMMARY

The present disclosure advantageously provides a method of treating or preventing bacterial biofilm formation during a breast augmentation surgical procedure comprising: providing a breast implant pocket at a surgical site in a patient and irrigating the pocket with a solution of hypochlorous acid. Additionally, the present disclosure provides a method of treating or preventing a *Ralstonia pickettii* infection during a breast augmentation procedure comprising: providing a breast implant pocket at a surgical site in a patient and irrigating the pocket with a solution of hypochlorous acid. In another embodiment, the present disclosure provides a method of preventing the occurrence of anaplastic large-cell lymphoma following a breast augmentation procedure comprising: providing a breast implant pocket at a surgical site in a patient and irrigating the pocket with a solution of hypochlorous acid.

In some embodiments, the present methods further comprise soaking a breast implant in a hypochlorous acid solution prior to inserting the implant into the pocket. In additional embodiments, the present methods further comprise irrigating the surgical site with a solution of hypochlorous acid after inserting the implant into the pocket.

In some embodiments, the hypochlorous acid has a concentration of about 0.01% to about 0.1%, about 0.01% to about 0.075%, about 0.02% to about 0.05%, about 0.02% to about 0.03%, or about 0.025%.

The present disclosure also provides a kit for treating or preventing biofilm formation during a breast augmentation procedure comprising: a container of a hypochlorous acid solution, a squirting or spraying device for administering the hypochlorous acid solution, and instructions for irrigating a breast implant pocket with the hypochlorous acid solution.

The present disclosure further provides hypochlorous acid solution for use in treating or preventing biofilm formation during a breast augmentation procedure, for use in treating or preventing a *Ralstonia pickettii* infection during a breast augmentation procedure, or for use in preventing the occurrence of anaplastic large-cell lymphoma following a breast augmentation surgical procedure

### BRIEF DESCRIPTION OF THE DRAWINGS

FIG. 1 is a graph summarizing the time kill assay results against *Ralstonia pickettii* ATCC 2711 using several different anti-microbial agents at clinical concentrations with a Mentor smooth implant.
FIG. 2 is a graph summarizing the time kill assay results against *Ralstonia pickettii* ATCC 2711 using several different anti-microbial agents at clinical concentrations with a Mentor siltex implant.
FIG. 3 is a graph summarizing the time kill assay results against *Ralstonia pickettii* ATCC 2711 using several different anti-microbial agents at clinical concentrations with an Allergan Biocell implant.
FIG. 4 depicts the average LOG CFU/mL curves against *Ralstonia pickettii* ATCC 2711 using several different anti-microbial agents at clinical concentrations with a Mentor smooth implant.
FIG. 5 depicts the average LOG CFU/mL curves against *Ralstonia pickettii* ATCC 2711 using several different anti-microbial agents at clinical concentrations with a Mentor siltex implant.
FIG. 6 depicts the average LOG CFU/mL curves against *Ralstonia pickettii* ATCC 2711 using several different anti-microbial agents at clinical concentrations with an Allergan Biocell implant.
FIG. 7 is a graph summarizing the time kill assay results against *Ralstonia pickettii* ATCC 2711 using several different anti-microbial agents at CT50 concentration with a Mentor smooth implant.
FIG. 8 is a graph summarizing the time kill assay results against *Ralstonia pickettii* ATCC 2711 using several different anti-microbial agents at CT50 concentration with a Mentor siltex implant.
FIG. 9 is a graph summarizing the time kill assay results against *Ralstonia pickettii* ATCC 2711 using several different anti-microbial agents at CT50 concentration with an Allergan Biocell implant.
FIG. 10 is a graph summarizing the time kill assay results against *Ralstonia pickettii* biofilm on a Mentor smooth implant using several different anti-microbial agents at clinical concentrations.
FIG. 11 is a graph summarizing the time kill assay results against *Ralstonia pickettii* biofilm on a Mentor siltex implant using several different anti-microbial agents at clinical concentrations.
FIG. 12 depicts the average LOG CFU/mL curves against *Ralstonia pickettii* biofilm on an Allergan Biocell implant using several different anti-microbial agents at clinical concentrations.
FIG. 13 depicts the average LOG CFU/mL curves against *Ralstonia pickettii* biofilm on a Mentor smooth implant using several different anti-microbial agents at clinical concentrations.
FIG. 14 depicts the average LOG CFU/mL curves against *Ralstonia pickettii* biofilm on a Mentor siltex implant using several different anti-microbial agents at clinical concentrations.
FIG. 15 is a graph summarizing the time kill assay results against *Ralstonia pickettii* biofilm on an Allergan Biocell implant using several different anti-microbial agents at clinical concentrations.

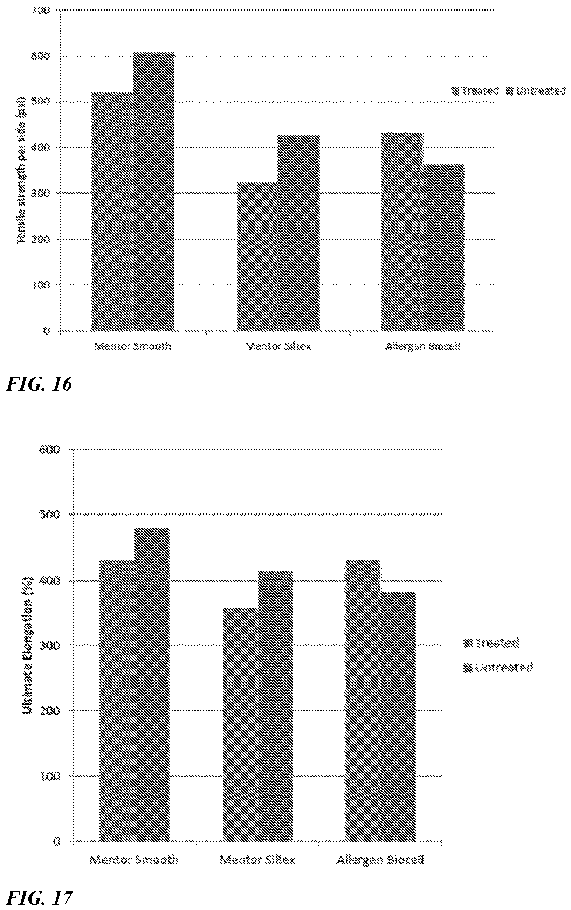

FIG. 16 is a graph depicting the tensile strength of three different implants that are untreated or treated with HOCl.

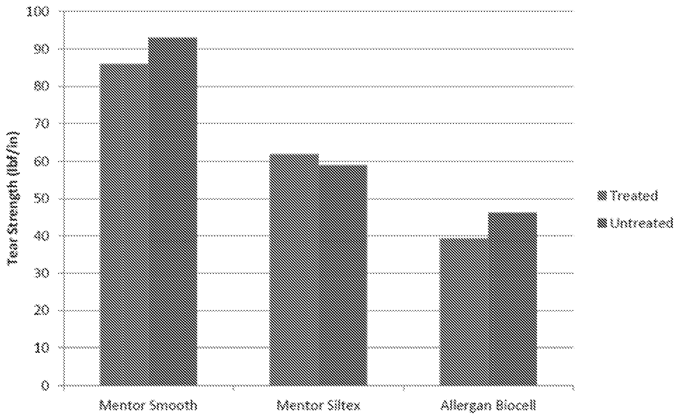

FIG. 17 is a graph depicting the percent elongation of three different implants that are untreated or treated with HOCl.
FIG. 18 is a graph depicting the tear strength of three different implants that are untreated or treated with HOCl.

### DETAILED DESCRIPTION

Breast implants are widely used in both reconstructive and aesthetic surgery, and strategies to reduce their contamination should be more widely studied and practiced. Hypochlorous acid is effective at reducing *Ralstonia pickettii* bacterial numbers and at disrupting the polysaccharide and protein matrix within the biofilm model. Hypochlorous acid may assist in the management of *Ralstonia pickettii* associated chronically infected wound sites resulting from reconstructive and aesthetic surgery by decreasing the Ralstonia pickettii bacterial numbers and by preventing and also penetrating and disrupting preformed polysaccharide/protein matrix of wound pathogen biofilms.

### EXAMPLES

We performed an *in vitro* bactericidal serial time-kill assay against Ralstonia *pickettii* ATCC 27511 with saline, Hypochlorous Solution (HOCL), povidone, chlorhexidine and triple antibiotic solutions in concentrations used clinically. We also performed a biofilm adherence assay against all solutions.

Time kill assays at clinical concentrations of 0.05% chlorhexidine gluconate, 10% povidone-iodine, triple-antibiotic solution (1 g Cefazolin, 80 mg Gentamicin and 50,000 U of Bacitracin in 500 mL saline), and stabilized 0.025% hypochlorous acid solution stabilized in amber glass were evaluated against Ralstonia pickettii ATCC 27511. Normal saline was used as the control solution. Three separate silicone implant types, Smooth Surface (Mentor Worldwide, Irvine, CA); Siltex (Mentor Worldwide, Irvine, CA); Biocell (Allergan plc, Dublin, Ireland) representing both smooth and textured surface implants were selected. Breast implant shells were thoroughly cleaned with distilled water and 70% alcohol and cut into uniform circles of 0.495 inch diameter using a punch. The cut implant shells were then dry heat sterilized before each experiment. Planktonic assays were performed after implants were soaked for 1, 5, 30, and 120 minute time points. *R. pickettii* was grown by streaking onto nutrient agar and incubating for 18 to 20 hours at 37°C. The organisms will be suspended in phosphate buffered saline (PBS) and adjusted to an optical density (OD) of 0.8 to 1.0 in phosphate buffered saline (PBS). This OD is equivalent to approximately 108 CFU/mL which is 0.5 McFarland. A total of 1 mL of test article was added to a glass test tube, 1 mL of saline served as a control to determine inoculum prior to treatment with the test article, and 10 µL of adjusted inoculum were added to each corresponding test tube to achieve a starting inoculum of 105 CFU/mL. At 1 min, 5 min, 30 mins, and 2 hour, 200 µL aliquots were taken from each test tube and added to Dey and Engley (D/E) neutralizing broth (Hardy Diagnostics, Santa Maria, CA) to neutralize the test article. A 200 µL aliquot was also taken from control tube and added to phosphate buffered saline.

Tenfold serial dilutions were performed for each sample tube using neutralizing buffer as the diluent. An amount of 100 µL of the appropriate dilutions of each sample were plated on a nutrient plate in duplicates and incubated overnight. The colonies were counted postincubation. If the test article is antimicrobial, there was a reduction in colony counts between the treatment groups. Data are evaluated by comparing the difference in CFU/mL for the 4 test articles and the blank control at 1 min, 5 min, 30 mins, and 2 hour time points.

### Cytotoxicity (CT50) Testing

In vitro cytotoxicity of test articles were tested against the Vero cell line (ATCC CCL- 1) using the cell proliferation kit Cell Titer 96 Aqueous One Solution Cell Proliferation Assay (Promega, Madison, WI). Briefly, cells were seeded into 96-well plates at a density of approximately 20,000 cells/well. The growth medium was RPMI 1640 medium (containing 10% Fetal Bovine Serum (FBS) and 2 mM L-glutamine and 100 IU/mL penicillin-100 µg/mL streptomycin). Cells wiere grown at 37°C with 5% CO2. After 24 hours, the media was removed from the wells by aspiration. The cells were then exposed to a series of 11 twofold dilutions of the test article in RPMI media for 24 hours at 37°C with 5% CO2 prior to measuring cell viability using the Cell Titer Proliferation assay.

### Biofilm Assay

Biofilm assays were performed after 2 to 3 mL of 105 CFU/mL bacterial inoculum was added to each tube containing the respective implants and placed into a shaking incubator (250-300 rpm) at 30°C for 24 hours, allowing for formation of biofilm on implants. After 24 hours' incubation, the implants having the biofilm were rinsed twice with Butterfields phosphate buffer. After the rinses, the implants were aseptically transferred to tubes containing 5 mL test articles for contact time points; 5 min and 2 hours. Postincubation, the implants were rinsed twice with Butterfields phosphate buffer, placed in 5 mL of sterile neutralizing buffer and sonicated at 50 to 60 Hz for 5 minutes. Tenfold serial dilutions were performed for each sample tube using neutralizing buffer as the diluent. A total of 100 µL of appropriate dilutions of each sample were plated on a nutrient agar plate in duplicates and incubated overnight. The colonies were counted postincubation. Data were evaluated by comparing the difference in CFU/mL for the implants and blank control at 5 min and 2 hours.

### RESULTS

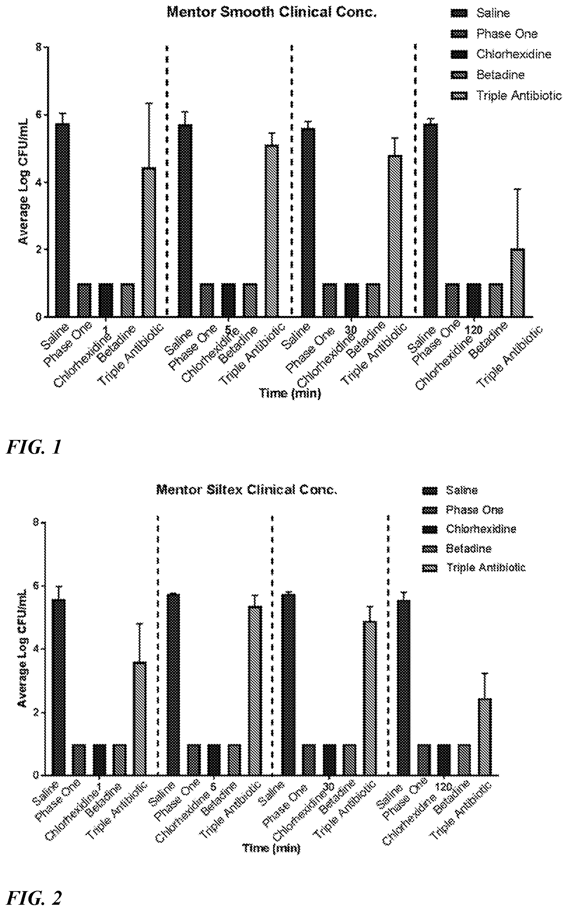

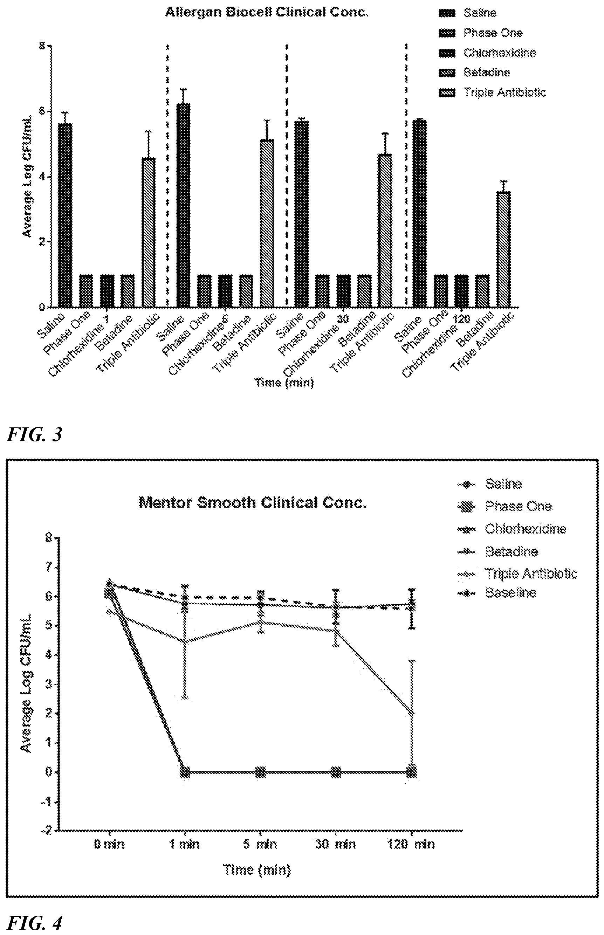

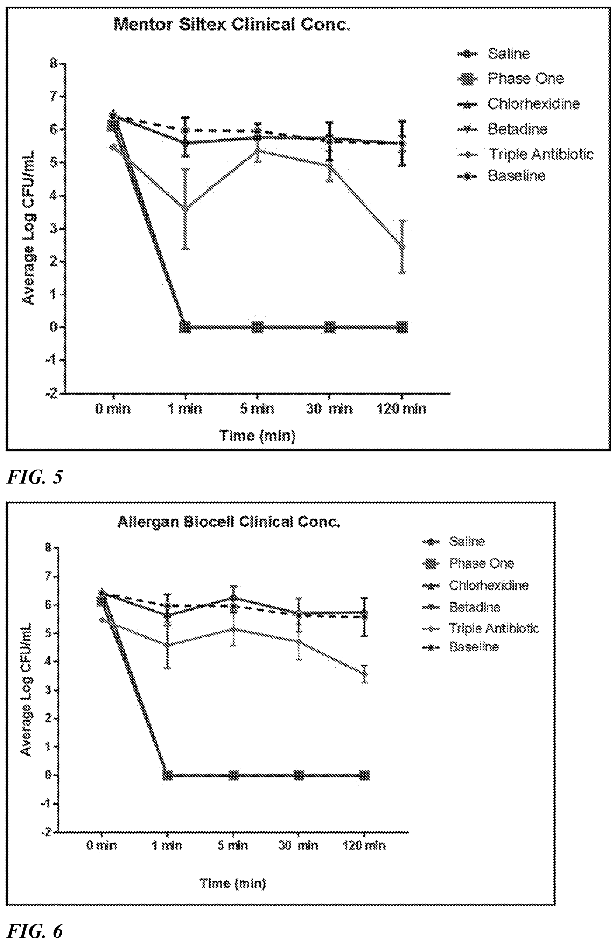

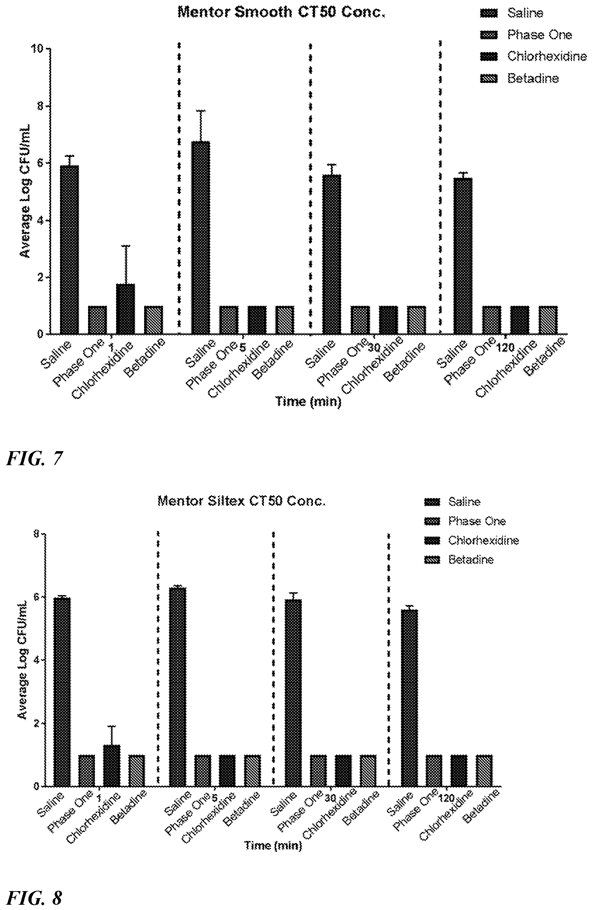

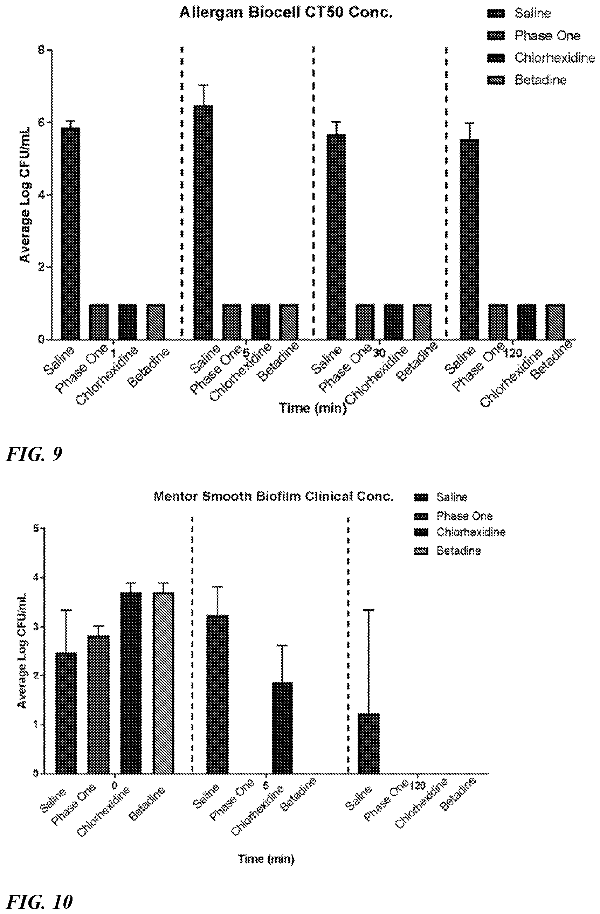

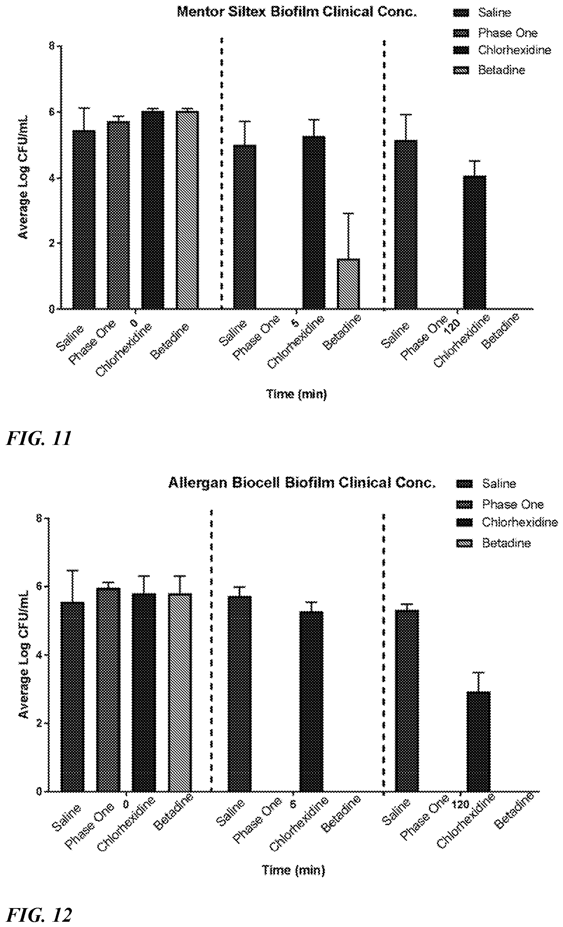

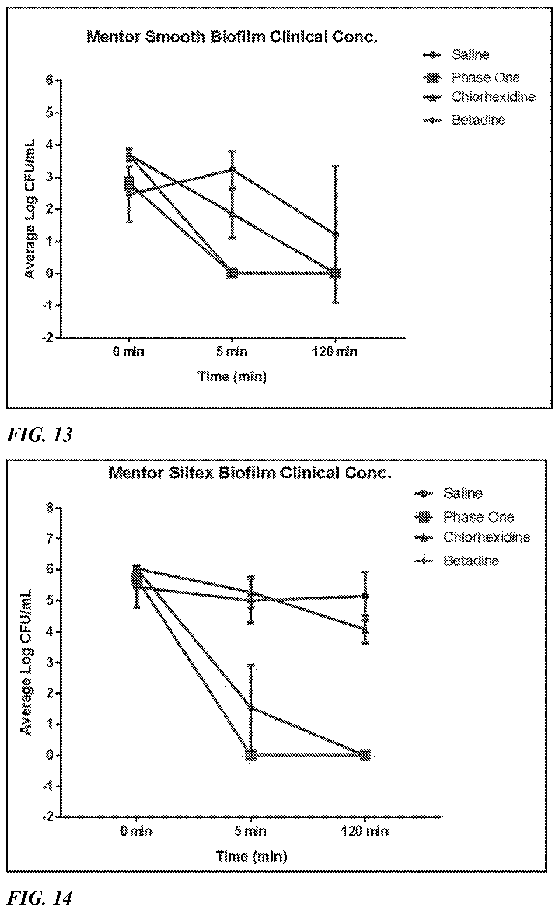

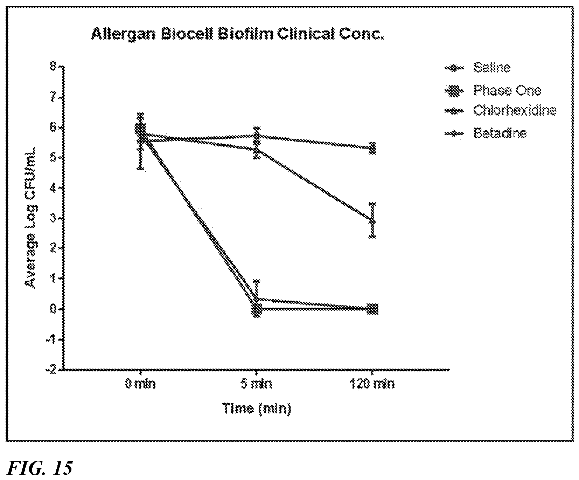

Figures 1-6 show the results of the test solutions and saline control against the planktonic form of the study bacteria on the three different types of implants. Triple antibiotic solution showed no effect on the study bacteria during planktonic assay and was therefore dropped from the study. All subsequent solutions showed total kill of planktonic bacteria at one minute soak times compared to saline control. Figures 7-9 show the results of the test solutions and saline control at CT50 concentrations. The results of these test solutions on the established *R*. *pickettii* biofilm are presented in Figures 10-15 based on the type of implant.

Biofilm assays showed differentiated penetration and impact of solutions on mature biofilm grown on the silicone implants. Saline control showed no significant effect on the biofilm for any of the implants, as anticipated. Chlorhexidine gluconate (Irrisept 0.05%, Irrimax Corporation, Lawrenceville, GA) did not have antibiofilm activity at Ralstonia biofilm-associated organisms at 5 minutes or at 2 hours for Siltex or Biocell, but did produce limited reduction at 5 minutes, and complete eradication at 2 hours for the smooth implant only. Povidone-Iodine 10% (Betadine, Purdue Pharma L.P., Stamford, CT) effectively eradicated biofilm on the smooth and Biocell implant at 5 minutes but required 2 hours of soak time to demonstrate complete biofilm eradication on Siltex. Pure HOCl (PhaseOne, Integrated Healing Technologies, Nashville, TN) demonstrated superior effectiveness in eradicating R. Pickettii biofilm on all three implant surfaces tested within the first five minute soak time. In this preliminary study, 0.025% hypochlorous acid in normal saline solution (1/32 dilution), stabilized in amber glass, successfully eradicated planktonic Ralstonia pickettii in 60 seconds and R. pickettii biofilm grown on all three silicone implants during an initial five minute soak time in vitro. Povidone iodine showed the potential of eradicating biofilm, however required 120 minute soak time compared to the five minute soak time of PhaseOne. Chlorhexidine gluconate 0.05% was unable to penetrate established biofilm after two hours and triple antibiotic was removed from the study due to inability to show impact on even planktonic forms of studied bacteria. Pure, 0.025% hypochlorous acid stabilized in amber glass (PhaseOne) may be the preferred antimicrobial solution to manage both planktonic bacteria and established biofilm phenotype bacteria associated with silicone breast implant infections, given its rapid action, chemical stability, and safety profile.

Thus, although there have been described particular embodiments of the present invention of a new and useful Method for Treatment and Prevention of Biofilm Formation During Breast Augmentation Procedures it is not intended that such references be construed as limitations upon the scope of this invention except as set forth in the following claim.

## Claims

1. A hypochlorous acid solution for use in treating or preventing biofilm formation during a breast augmentation procedure.

2. A hypochlorous acid solution for use in treating or preventing a *Ralstonia pickettii* infection during a breast augmentation procedure.

3. A hypochlorous acid solution for use in preventing the occurrence of anaplastic large-cell lymphoma following a breast augmentation surgical procedure

4. The hypochlorous acid solution for the use of claim 1, 2, or 3, wherein the hypochlorous acid has a concentration of about 0.01% to about 0.1% in the solution.

5. The hypochlorous acid solution for the use of claim 4, wherein the concentration is about 0.01% to about 0.075%.

6. The hypochlorous acid solution for the use of claim 4, wherein the concentration is about 0.02% to about 0.05%.

7. The hypochlorous acid solution for the use of claim 4, wherein the concentration is about 0.02% to about 0.03%.

8. The hypochlorous acid solution for the use of claim 4, wherein the concentration is about 0.025%.

9. A kit for use in treating or preventing biofilm formation during a breast augmentation procedure comprising:
a container of a hypochlorous acid solution,
a squirting or spraying device for administering the hypochlorous acid solution,
instructions for said treatment and prevention, which comprise the steps of providing a breast implant pocket at a surgical site in a patient and
irrigating the breast implant pocket with the hypochlorous acid solution.

10. A breast implant soaked in a hypochlorous acid solution.

## Patentansprüche

1. Hypochlorige Säurelösung zur Verwendung bei der Behandlung von oder Vorbeugung gegen Biofilmbildung während eines Brustvergrößerungsverfahrens.

2. Hypochlorige Säurelösung zur Verwendung bei der Behandlung von oder Vorbeugung gegen eine *Ralstonia pickettii-*Infektion während eines Brustvergrößerungsverfahrens.

3. Hypochlorige Säurelösung zur Verwendung bei der Vorbeugung gegen das Auftreten von anaplastischen großzelligen Lymphomen nach einem chirurgischen Eingriff zur Brustvergrößerung.

4. Hypochlorige Säurelösung zur Verwendung nach Anspruch 1, 2 oder 3,
wobei die hypochlorige Säure eine Konzentration von etwa 0,01% bis etwa 0,1% in der Lösung aufweist.

5. Hypochlorige Säurelösung zur Verwendung nach Anspruch 4,
wobei die Konzentration von etwa 0,01% bis etwa 0,075% beträgt.

6. Hypochlorige Säurelösung zur Verwendung nach Anspruch 4,
wobei die Konzentration von etwa 0,02% bis etwa 0,05% beträgt.

7. Hypochlorige Säurelösung zur Verwendung nach Anspruch 4,
wobei die Konzentration von etwa 0,02% bis etwa 0,03% beträgt.

8. Hypochlorige Säurelösung zur Verwendung nach Anspruch 4,
wobei die Konzentration etwa 0,025% beträgt.

9. Kit zur Verwendung bei der Behandlung von oder Vorbeugung gegen Biofilmbildung während eines Brustvergrößerungsverfahrens, umfassend:
einen Behälter mit einer hypochlorigen Säurelösung,
eine Spritz- oder Sprühvorrichtung zum Verabreichen der hypochlorigen Säurelösung,
Anweisungen für die Behandlung und Vorbeugung, die Schritte des Bereitstellens einer Brustimplantattasche an einer Operationsstätte bei einem Patienten und des Spülens der Brustimplantattasche mit der hypochlorigen Säurelösung umfassen.

10. Brustimplantat, das in einer hypochlorigen Säurelösung getränkt ist.

## Revendications

1. Solution d'acide hypochloreux pour utilisation dans le traitement ou la prévention de la formation de biofilm pendant une procédure d'augmentation mammaire.

2. Solution d'acide hypochloreux pour utilisation dans le traitement ou la prévention d'une infection à *Ralstonia pickettii* pendant une procédure d'augmentation mammaire.

3. Solution d'acide hypochloreux pour utilisation dans la prévention de la survenue de lymphome anaplasique à grandes cellules suite à une procédure chirurgicale d'augmentation mammaire.

4. Solution d'acide hypochloreux pour l'utilisation selon la revendication 1, 2 ou 3, dans laquelle l'acide hypochloreux a une concentration d'environ 0,01 % à environ 0,1 % dans la solution.

5. Solution d'acide hypochloreux pour l'utilisation selon la revendication 4, dans laquelle la concentration est d'environ 0,01 % à environ 0,075 %.

6. Solution d'acide hypochloreux pour l'utilisation selon la revendication 4, dans laquelle la concentration est d'environ 0,02 % à environ 0,05 %.

7. Solution d'acide hypochloreux pour l'utilisation selon la revendication 4, dans laquelle la concentration est d'environ 0,02 % à environ 0,03 %.

8. Solution d'acide hypochloreux pour l'utilisation selon la revendication 4, dans laquelle la concentration est d'environ 0,025 %.

9. Kit pour utilisation dans le traitement ou la prévention de la formation de biofilm pendant une procédure d'augmentation mammaire comprenant :
un contenant d'une solution d'acide hypochloreux,
un dispositif de giclage ou de pulvérisation pour l'administration de la solution d'acide hypochloreux,
des instructions pour lesdits traitement et prévention, qui comprennent les étapes de fourniture d'une poche d'implant mammaire sur un site chirurgical d'un patient et d'irrigation de la poche d'implant mammaire avec la solution d'acide hypochloreux.

10. Implant mammaire imprégné d'une solution d'acide hypochloreux.
